(19) Europäisches Patentamt
European Patent Office
Office européen des brevets

(11) Publication number: **0 189 960 B1**

# (12) EUROPEAN PATENT SPECIFICATION

(45) Date of publication of patent specification: **24.04.91**

(51) Int. Cl.5: **C07D 277/30**, C07D 277/20, A01N 43/78

(21) Application number: **86200094.0**

(22) Date of filing: **20.01.86**

The file contains technical information submitted after the application was filed and not included in this specification

## (54) Novel insecticidal 1,3-thiazole derivatives.

(30) Priority: **01.02.85 US 697546**

(43) Date of publication of application:
**06.08.86 Bulletin 86/32**

(45) Publication of the grant of the patent:
**24.04.91 Bulletin 91/17**

(84) Designated Contracting States:
**AT BE CH DE FR GB IT LI LU NL SE**

(56) References cited:
**GB-A- 2 051 052**
**GB-A- 2 051 060**
**US-A- 4 153 705**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Kollmeyer, Willy Dietrich**
**1008 Stratford Lane**
**Modesto California 95350(US)**

(74) Representative: **Bennett, David Arthur Horder et al**
**4, York Road**
**London SE1 7NA(GB)**

EP 0 189 960 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to novel insecticidal 1,3-thiazole derivatives.

U.S. patents 4,153,705, 4,297,361 and 4,320,125 disclose that certain 1,3-thiazole compounds are useful as insecticides. However, there continues to be a need for new insecticides, particularly ones effective against Lepidoptera species.

The present invention provides a compound of the general formula

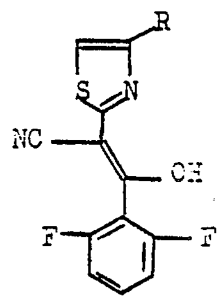

(I)

or a tautomer thereof, wherein R is an alkyl group containing 2 to 6 carbon atoms optionally substituted by one or more halogen atoms, a cycloalkyl group containing 3 to 6 ring carbon atoms optionally substituted by one or more alkyl groups containing 1 or 2 carbon atoms, an aryl or aralkyl group containing 6 to 10 carbon atoms optionally ring-substituted by one or more halogen atoms or alkyl groups containing 1 or 2 carbon atoms, or an alkoxycarbonylalkyl group in which each alkyl portion contains from 1 to 6 carbon atoms. The compounds are useful as insecticides.

It will be appreciated that the compounds of the invention may exist in tautomeric forms represented by the formulae:

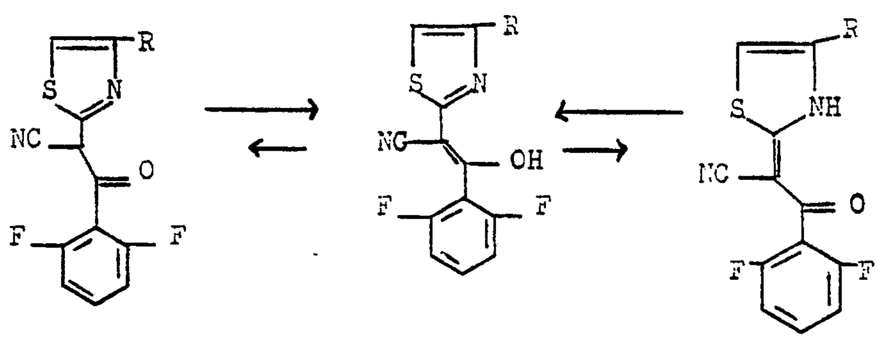

I(a) (I) I(b)

It is believed that the compounds exist predominantly in the enol form (I), but may adopt either of the tautomeric forms I(a) or I(b) under certain conditions. The invention should be understood to include all three tautomeric forms and mixtures thereof.

Preferably R represents an alkyl group containing 2 to 6 carbon atoms, especially a branched-chain alkyl group. Especially preferred are compounds in which R is a tertiary-butyl or a 1,1-dimethylpropyl group.

The invention also provides a process for the preparation of a compound according to the invention, which comprises reacting a compound of the general formula

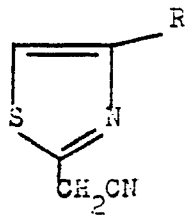

(II)

in which R has the meaning given for the general formula I, with 2,6-difluorobenzoyl chloride, in the presence of a base. Suitable bases include alkali metal alkoxides, for example sodium ethoxide, and amines, for example triethylamine, pyridine or alkylpyridines. The reaction is preferably carried out at a temperature in the range of 0 to 100 ° C, in the presence of a solvent, for example a hydrocarbon or chlorinated hydrocarbon. Room temperature is often convenient.

The compounds of formula II may be prepared by cyclization of $NH_2$-C(S)$CH_2$CN with RC(O)$CH_2$Hal in which R is defined as for formula I, and Hal is halogen, preferably bromine or chlorine, in the presence of a base, such as ethanolic potassium hydroxide.

Typical examples of compounds of the invention include the specific compounds of formula I wherein R is ethyl, propyl, isopropyl, butyl, isobutyl, secondary-butyl, cyclobutyl, 1-methylcyclobutyl, cyclopentyl, cyclohexyl, 2,6-dimethylphenyl, 2,4,6-trimethylphenyl, 2-chlorophenyl, 2,4-dichlorophenyl, 2,6-dichlorophenyl, 2,6-difluorophenyl, or 2-fluorophenyl and the like.

The compounds of the invention exhibit insecticidal activity, for example against Lepidoptera and against beetles including Colorado beetles. Accordingly, the invention provides an insecticidal composition which comprises a compound according to the invention together with a carrier. The invention further provides a method of combating insects at a locus, which comprises treating the locus with a compound or composition according to the invention. The locus may for example be a crop area, or stored agricultural produce. The effective dosage will of course vary depending on the intended use, but in general a dosage of 0.0001% to 2% by weight of active compound based on the total weight of formulation applied, is suitable.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably composition according to the invention contain 0.5 to 95% by weight of active ingredient.

Suitable sodid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts or polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p -octylphenol or p -octylcresol, with ethylene oxide and/or propylene oxide; sulphates or sulphonates of these condensation

products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Of particular interest in current practice are the water-dispersible granular formulations. These are in the form of dry, hard granules that are essentially dust-free, and are resistant to attrition on handling, thus minimising the formation of dust. On contact with water, the granules readily disintegrate to form stable suspensions of the particles of active material. Such formulations contain 90% or more by weight of finely divided active material, 3-7% by weight of a blend of surfactants, which act as wetting, dispersing, suspending and binding agents, and 1-3% by weight of a finely divided carrier, which acts as a resuspending agent. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The following Examples illustrate the invention. The identity of the products, including intermediates, was confirmed by elemental, infra red and NMR analyses as necessary. Examples 1 to 9 illustrate the preparation of intermediates of formula II, while Examples 10 to 16 illustrate compounds of the formula I.

Example 1: 4-(1,1-Dimethylpropyl)-1,3-thiazole-2-acetonitrile

To a solution of 10.72g of 85% potassium hydroxide in 200ml ethanol was added 15.56g of 2-cyanothioacetamide. After stirring at ambient temperature for one hour, the reaction mixture was cooled with an ice bath, treated dropwise with 30.00g of 1-bromo-3,3-dimethyl-2-pentanone and stirred at ambient temperature overnight. The reaction mixture was filtered, and the solvent was removed under reduced pressure. The resulting residue dissolved in methylene chloride was passed through a column of Florasil (Trade Mark) to give a brown oil. Further purification by distillation gave 19.74g of the desired product as a yellow oil.

Example 2: 4-(1,1-Dimethylethyl)-1,3-thiazole-2-acetonitrile

To a solution of 16.5g of 85% KOH in 250ml ethanol was added 25.0g of 2-cyanothioacetamide. 44.8g of bromopinacolone was then added dropwise over 5 minutes. The reaction temperature spontaneously climbed to 65°C and a white solid began to deposit. The mixture was stirred and heated at reflux for 1 hour, cooled, poured into 1000ml water, and extracted with diethyl ether. The extract was washed with

brine, dried over $MgSO_4$, and concentrated. Distillation of the residual oil gave 23.3g of the desired product as a light yellow oil b.p. 90-95° C (1mm Hg).

Examples 3 to 9

Following procedures similar to those described in Examples 1 and 2 above, the following compounds of formula II were prepared.

Table I: Compounds of Formula II

| Example No. | R in the Formula II | b.p.°C (mm Hg) or m.p.°C |
|---|---|---|
| 3 | o-tolyl | 160-165 (0.02) |
| 4 | 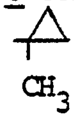 | Not measured |
| 5 | phenyl | 49-52 |
| 6 | $C(CH_3)_2CH_2Cl$ | Not measured |
| 7 | $C(CH_3)_2CO_2CH_3$ | Not measured |
| 8 | $C(CH_3)_2CO_2C_2H_5$ | 125 (0.05) |
| 9 | $C(CH_3)2CO_2i\text{-}C_3H_7$ | Not measured |

Examole 10 Alpha-[hydroxy(2,6-difluorophenyl)methylene]-4-(1,1-dimethylpropyl)-1,3-thiazole-2-acetonitrile

A stirred solution of 3.5g of the thiazole of Example 1 above, 0.2g of 4-dimethylaminopyridine and 3ml triethylamine in 100ml benzene at ambient temperature was treated dropwise with 3.17g of 2,6-difluorobenzoyl chloride. The reaction mixture was stirred overnight at room temperature, diluted with methylene chloride, washed with water, dried ($MgSO_4$), and stripped to give 9.50g of a brown oil. Flash chromatography with methylene chloride on silica gel, followed by trituration with diethyl ether yielded 0.43g of the desired product as yellow crystals m.p. 195-196.5° C.

Example 11 Alpha-[hydroxy(2,6-difluorophenyl)methylene]-4-(1,1-dimethylethyl)-1,3-thiazole-2-acetonitrile

A) Acylation with Excess Acid Chloride. To a mixture of 4.0g of the thiazole of Example 2 above, 5ml of triethylamine and 0.2g of 4-dimethylaminopyridine in 100ml of toluene was added dropwise 15.8g of 2,6-difluorobenzoyl chloride. After stirring overnight at room temperature, the mixture was diluted with diethyl ether and washed with water. Removal of solvent gave a multicomponent mixture as a black oil. Chromatography on a silica gel column using hexane:diethyl ether (70:30 by volume) as eluent gave 1.8g of a white solid identified as 2,6-difluorobenzoic acid anhydride. The column was then stripped with methylene chloride. This eluent, when stripped and triturated with diethyl ether, led to 1.0g of the desired product as a tan powder, m.p. 207-209° C.

B) Acylation with One Equivalent of Acid Chloride. A mixture of 2.0g of the thiazole of Example 2, and 2.1ml of triethylamine in 25ml of benzene was treated with 2.0g of 2,6-difluorobenzoyl chloride. After stirring overnight at room temperature with exclusion of ambient atmosphere, a catalytic amount of 4-dimethylaminopyridine, 0.2g, was added, and stirring was continued for an other 24 hours. At this point the mixture was diluted to 100ml with methylene chloride and washed with water. The organic layer was dried ($MgSO_4$) and concentrated. The resultant brown oil was taken up in 100ml methanol, treated with 2

drops of concentrated $H_2SO_4$, and refluxed for 5 hours. When this solution was cooled and poured into 200ml of water, the product crystallized. Filtration afforded 2.9g of the desired product; m.p. 207-208.5 °C.

Examples 12 to 16

Following procedures similar to those described in Examples 10 and 11 above, the following compounds of Formula I were prepared.

TABLE II: Compounds of the Formula I

| Example No. | R in the General Formula I | m.p., °C |
| --- | --- | --- |
| 12 | o-tolyl | 192-193 |
| 13 | 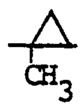 | 205-207 |
| 14 | phenyl | 205-207 |
| 15 | $C(CH_3)_2CH_2Cl$ | 183-186 |
| 16 | $C(CH_3)_2CO_2C_2H_5$ | >280 |

Example 17 Insecticidal Activity

Activity of compounds of the invention with respect to insects was determined as follows.

Third instar corn earworm larvae (Heliothis zea (Boddie)) were tested by spraying broad bean plants with dilutions of an acetone solution of the test compound in water containing an emulsifier. Immediately after spraying, 5 larvae were transferred to the plant and held for 44-46 hours, at which time the dead and moribund larvae were counted. The tests were conducted employing several different dosage rates for each test

In each test, an identical test was conducted using Parathion as a standard for comparison.

In each instance, the toxicity of the test compound was compared to that of Parathion, the relative toxicity of the test compound then being expressed in terms of the relationship between the amount of the test compound and the amount of the standard pesticide required to produce the same percentage (50%) of mortality in the test insects. By assigning the standard pesticide an arbitrary rating of 100, the toxicity of the test compound was expressed in terms of the Toxicity Index, which compares the toxicity of the test compound of the invention with that of the standard pesticide. That is to say, a test compound having a Toxicity Index of 50 would be half as active, while one having a Toxicity Index of 200 would be twice as active, as the standard pesticide. The results are set forth in Table III.

TABLE III: Insecticidal Activity

| Example No. | Toxicity Index |
|---|---|
| 10 | 219 |
| 11 | 329 |
| 12 | 128 |
| 13 | 65 |
| 14 | 40 |
| 15 | 41 |
| 16 | 2 |

## Claims

1. A compound of the general formula

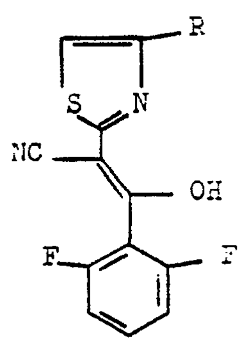

(I)

or a tautomer thereof, wherein R is an alkyl group containing 2 to 6 carbon atoms optionally substituted by one or more halogen atoms, a cycloalkyl group containing 3 to 6 ring carbon atoms optionally substituted by one or more alkyl groups containing 1 or 2 carbon atoms, an aryl or aralkyl group containing 6 to 10 carbon atoms optionally ring-substituted by one or more halogen atoms or alkyl groups containing 1 or 2 carbon atoms, or an alkoxycarbonylalkyl group in which each alkyl portion contains from 1 to 6 carbon atoms.

2. A compound as claimed in claim 1 wherein R is an alkyl group containing 2 to 6 carbon atoms.

3. A compound as claimed in claim 2 wherein R is a branched-chain alkyl group.

4. A compound as claimed in claim 3 wherein R is a tertiary-butyl or 1,1-dimethylpropyl group.

5. A compound as claimed in claim 1 and named in any one of Examples 10 to 16 herein.

6. A process for the preparation of a compound as claimed in claim 1, which comprises reacting a compound of the general formula

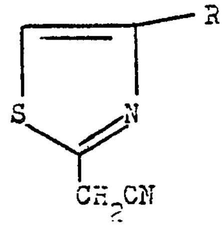

(II)

in which R has the meaning given in claim 1, with 2,6-difluorobenzoyl chloride, in the presence of a base.

7. An insecticidal composition which comprises a compound as claimed in any one of claims 1 to 5 together with a carrier.

8. A composition as claimed in claim 7, which comprises at least two carriers, at least one of which is a surface-active agent.

9. A method of combating insects at a locus, which comprises treating the locus with a compound as claimed in any one of claims 1 to 5 or a composition as claimed in either claim 7 or claim 8.

Claims for the following Contracting State: AT

1. A process for the preparation of a compound of the general formula

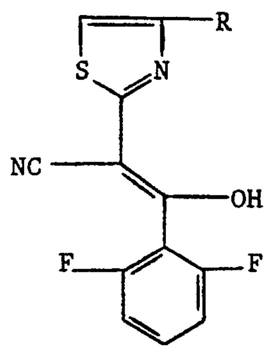

(I)

or a tautomer thereof, wherein R is an alkyl group containing 2 to 6 carbon atoms optionally substituted by one or more halogen atoms, a cycloalkyl group containing 3 to 6 ring carbon atoms optionally substituted by one or more alkyl groups containing 1 or 2 carbon atoms, an aryl or aralkyl group containing 6 to 10 carbon atoms optionally ring-substituted by one or more halogen atoms or alkyl groups containing 1 or 2 carbon atoms, or an alkoxycarbonylalkyl group in which each alkyl portion contains from 1 to 6 carbon atoms, which comprises reacting a compound of the general formula

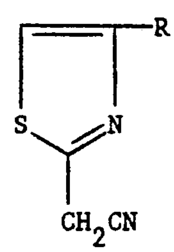

(II)

in which R has the meaning given hereinbefore, with 2,6-difluorobenzoyl chloride, in the presence of a base.

2. A process as claimed in claim 1 wherein R is an alkyl group containing 2 to 6 carbon atoms.

3. A process as claimed in claim 2 wherein R is a branched-chain alkyl group.

4. A process as claimed in claim 3 wherein R is a tertiary-butyl or 1,1-dimethylpropyl group.

5. An insecticidal composition which comprises a compound of the general formula

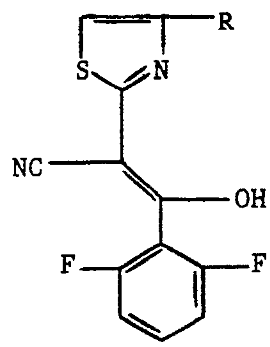

(I)

or a tautomer thereof, wherein R is an alkyl group containing 2 to 6 carbon atoms optionally substituted by one or more halogen atoms, a cycloalkyl group containing 3 to 6 ring carbon atoms optionally substituted by one or more alkyl groups containing 1 or 2 carbon atoms, an aryl or aralkyl group containing 6 to 10 carbon atoms optionally ring-substituted by one or more halogen atoms or alkyl groups containing 1 or 2 carbon atoms, or an alkoxycarbonylalkyl group in which each alkyl portion contains from 1 to 6 carbon atoms, together with a carrier.

6. A composition as claimed in claim 5, which comprises at least two carriers, at least one of which is a surface-active agent.

7. A method of combating insects at a locus, which comprises treating the locus with a composition as claimed in either claim 5 or claim 6.

**Revendications**

1. Un composé de la formule générale

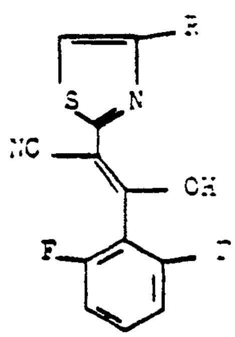

(I)

ou un tautomère d'un tel composé, où R est un groupe alcoyle contenant 2 à 6 atomes de carbone éventuellement substitué par un ou plusieurs atomes d'halogènes, un groupe cycloalcoyle contenant 3 à 6 atomes de carbone dans le cycle éventuellement substitué par un ou plusieurs groupes alcoyle contenant 1 ou 2 atomes de carbone, un groupe aryle ou aralcoyle contenant 6 à 10 atomes de carbone éventuellement substitué au noyau par un ou plusieurs atomes d'halogènes ou groupes alcoyle contenant 1 ou 2 atomes de carbone ou un groupe alcoxycarbonylalcoyle dans lequel chaque portion alcoyle contient de 1 à 6 atomes de carbone.

2. Un composé selon la revendication 1, dans lequel R est un groupe alcoyle contenant 2 à 6 atomes de

carbone.

3. Un composé selon la revendication 2, dans lequel R est un groupe alcoyle à chaîne ramifiée.

4. Un composé selon la revendicaticn 3, dans lequel R est un groupe tertiobutyle ou 1,1-diméthylpropyle.

5. Un composé selon la revendication 1 et nommé dans l'un quelconque des exemples 10 à 16 ci-dessus.

6. Un procédé de préparation d'un composé tel que défini dans la revendication 1, qui comprend la réaction d'un composé de la formule générale

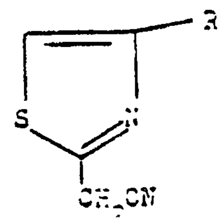

(II)

où R a la signification indiquée dans la revendication 1, avec le chlorure de 2,6-difluorobenzoyle, en présence d'une base.

7. Une composition insecticide qui comprend un composé selon l'une quelconque des revendications 1 à 5 en même temps qu'un véhicule.

8. Une composition selon la revendication 7, qui comprend au moins deux véhicules, dont au moins un est un agent tensio-actif.

9. Une méthode de lutte contre des insectes en un lieu, qui comprend le traitement du lieu avec un composé selon l'une quelconque des revendications 1 à 5 ou avec une composition selon la revendication 7 ou la revendication 8.

Revendícations pour l'Etat contractant suivant: AT

1. Un procédé de préparation d'un composé de la formule générale

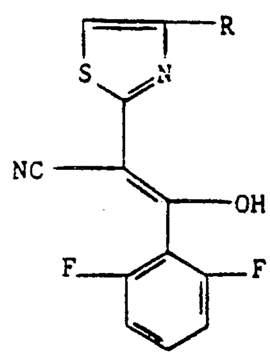

(I)

ou d'un tautomère d'un tel composé, où R est un groupe alcoyle contenant 2 à 6 atomes de carbone éventuellement substitué par un ou plusieurs atomes d'halogènes, un groupe cycloalcoyle contenant 3 à 6 atomes de carbone dans le cycle éventuellement substitué par un ou plusieurs groupes alcoyle contenant 1 ou 2 atomes de carbone, un groupe aryle ou aralcoyle contenant 6 à 10 atomes de carbone éventuellement substitué au noyau par un ou plusieurs atomes d'halogènes ou groupes alcoyle contenant 1 ou 2 atomes de carbone ou un groupe alcoxycarbonylalcoyle dans lequel chaque portion alcoyle contient de 1 à 6 atomes de carbone, qui comprend la réaction d'un composé de la formule générale

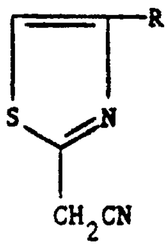

(II)

où R a la signification indiquée ci-dessus, avec le chlorure de 2,6-difluorobenzoyle, en présence d'une base.

2. Un procédé selon la revendication 1, dans lequel R est un groupe alcoyle contenant 2 à 6 atomes de carbone.

3. Un procédé selon la revendication 2, dans lequel R est un groupe alcoyle à chaîne ramifiée.

4. Un procédé selon la revendication 3, dans lequel R est un groupe tertiobutyle ou 1,1-diméthylpropyle.

5. Une composition insecticide qui comprend un composé de la formule générale

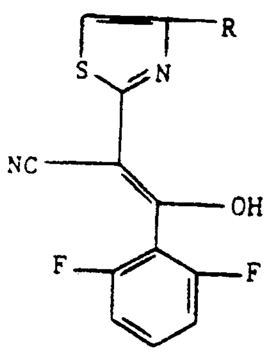

(I)

ou un tautomère d'un tel composé, où R est un groupe alcoyle contenant 2 à 6 atomes de carbone éventuellement substitué par un ou plusieurs atomes d'halogènes, un groupe cycloalcoyle contenant 3 à 6 atomes de carbone dans le cycle éventuellement substitué par un ou plusieurs groupes alcoyle contenant 1 ou 2 atomes de carbone, un groupe aryle ou aralcoyle contenant 6 à 10 atomes de carbone éventuellement substitué au noyau par un ou plusieurs atomes d'halogènes ou groupes alcoyle contenant 1 ou 2 atomes de carbone ou un groupe alcoxycarbonylalcoyle dans lequel chaque portion alcoyle contient 1 à 6 atomes de carbone, en même temps qu'un véhicule.

6. Une composition selon la revendication 5, qui comprend au moins deux véhicules, dont au moins un est un agent tensio-actif.

7. Une méthode de lutte contre des insectes en un lieu, qui comprend le traitement du lieu avec une composition selon la revendication 5 ou la revendication 6.

## Ansprüche

1. Verbindung der allgemeinen Formel

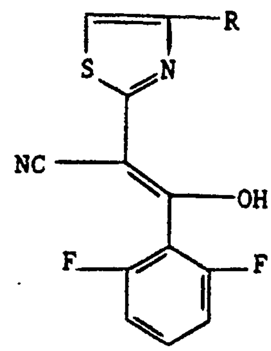

(I)

oder ein Tautomer hievon, worin R eine 2 bis 6 Kohlenstoffatome enthaltende Alkylgruppe, die gegebenenfalls durch ein oder mehrere Halogenatome substituiert ist, eine 3 bist 6 Ringkohlenstoffatome enthaltende Cycloalkylgruppe, die gegebenenfalls durch ein oder mehrere Alkylgruppen mit ein oder zwei Kohlenstoffatomen substituiert ist, eine 6 bis 10 Kohlenstoffatome enthaltende Aryl- oder Aralkylgruppe, die gegebenenfalls durch ein oder mehrere Halogenatome oder Alkylgruppen mit ein oder zwei Kohlenstoffatomen ringsubstituiert ist, oder eine Alkoxycarbonylalkylgruppe bedeutet, worin jeder Alkylteil 1 bis 6 Kohlenstoffatome enthält.

2. Verbindung nach Anspruch 1, worin R eine 2 bis 6 Kohlenstoffatome enthaltende Alkylgruppe bedeutet.

3. Verbindung nach Anspruch 2, worin R eine verzweigtkettige Alkylgruppe darstellt.

4. Verbindung nach Anspruch 3, worin R eine tertiäre Butylgruppe oder eine 1,1-Dimethylpropylgruppe darstellt.

5. Eine Verbindung, wie in Anspruch 1 beansprucht und in einem der vorliegenden Beispiele 10 bis 16 benannt.

6. Verfahren zur Herstellung einer Verbindung, wie in Anspruch 1 beansprucht, welches Verfahren ein Umsetzen einer Verbindung der allgemeinen Formel

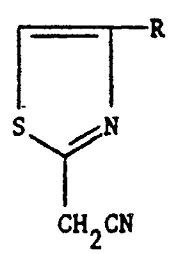

(II) ,

worin R die in Anspruch 1 angegebene Bedeutung aufweist, mit 2,6-Difluorbenzoylchlorid in Gegenwart einer Base umfaßt.

7. Eine insektizide Zusammensetzung, welche eine Verbindung, wie in einem der Ansprüche 1 bis 5 beansprucht, zusammen mit einem Träger umfaßt.

8. Zusammensetzung nach Anspruch 7, welche wenigstens zwei Träger umfaßt, von denen wenigstens einer ein oberflächenaktives Mittel ist.

9. Verfahren zur Bekämpfung von Insekten an einem Ort, welches ein Behandeln des Ortes mit einer Verbindung, wie in einem der Ansprüche 1 bis 5 beansprucht, oder mit einer Zusammensetzung, wie in Anspruch 7 oder Anspruch 8 beansprucht, umfaßt.

Patentansprüche für folgenden Vertragsstaat: AT

1. Verfahren zur Herstellung einer Verbindung der allgemeinen Formel

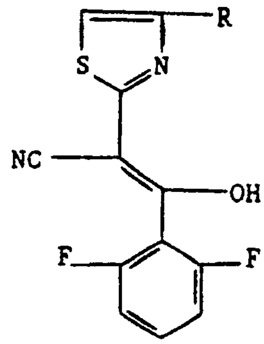

(I)

oder eines Tautomers hievon, worin R eine 2 bis 6 Kohlenstoffatome enthaltende Alkylgruppe, die gegebenenfalls durch ein oder mehrere Halogenatome substituiert ist, eine 3 bis 6 Ringkohlenstoffatome enthaltende Cycloalkylgruppe, die gegebenenfalls durch eine oder mehrere Alkylgruppen mit ein oder zwei Kohlenstoffatomen substituiert ist, eine 6 bis 10 Kohlenstoffatome enthaltende Aryl- oder Aralkylgruppe, die gegebenenfalls durch ein oder mehrere Halogenatome oder Alkylgruppen mit ein oder zwei Kohlenstoffatomen ringsubstituiert ist, oder eine Alkoxycarbonylalkylgruppe darstellt, worin jeder Alkylteil 1 bis 6 Kohlenstoffatome enthält, welches Verfahren ein Umsetzen einer Verbindung der allgemeinen Formel

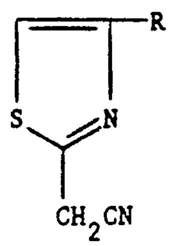

(II) ,

worin R die vorstehend angegebene Bedeutung aufweist, mit 2,6-Di-fluorbenzoylchlorid in Gegenwart einer Base umfaßt.

**2.** Verfahren nach Anspruch 1, worin R eine 2 bis 6 Kohlenstoffatome enthaltende Alkylgruppe darstellt.

**3.** Verfahren nach Anspruch 2, worin R eine verzweigtkettige Alkylgruppe darstellt.

**4.** Verfahren nach Anspruch 3, worin R eine tertiäre Butylgruppe oder eine 1,1-Dimethylpropylgruppe darstellt.

**5.** Insektizide Zusammensetzung, welche eine Verbindung der allgemeinen Formel

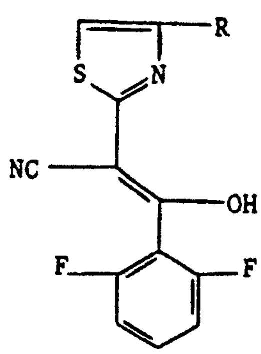

(I)

oder ein Tautomer hievon, worin R eine 2 bis 6 Kohlenstoffatome enthaltende Alkylgruppe, die gegebenenfalls durch ein oder mehrere Halogenatome substituiert ist, eine 3 bis 6 Ringkohlenstoffatome enthaltende Cycloalkylgruppe, die gegebenenfalls durch ein oder mehrere Alkylgruppen mit ein oder zwei Kohlenstoffatomen substituiert ist, eine 6 bis 10 Kohlenstoffatome enthaltende Aryl- oder Aralkylgruppe, die gegebenenfalls durch ein oder mehrere Halogenatome oder Alkylgruppen mit ein oder zwei Kohlenstoffatomen ringsubstituiert ist, oder eine Alkoxycarbonylalkylgruppe bedeutet, worin jeder Alkylteil 1 bis 6 Kohlenstoffatome enthält, zusammen mit einem Träger umfaßt.

6. Zusammensetzung nach Anspruch 5, welche wenigstens zwei Träger umfaßt, von denen wenigstens einer ein oberflächenaktives Mittel ist.

7. Verfahren zur Bekämpfung von Insekten an einem Ort, welches ein Behandeln des Ortes mit einer Zusammensetzung, wie in Anspruch 5 oder Anspruch 6 beansprucht, umfaßt.